(19) Europäisches Patentamt
European Patent Office
Office européen des brevets

(11) **EP 3 556 287 B1**

(12) **FASCICULE DE BREVET EUROPEEN**

(45) Date de publication et mention
de la délivrance du brevet:
**13.10.2021 Bulletin 2021/41**

(51) Int Cl.:
*A61B 5/06* (2006.01)          *A61B 5/11* (2006.01)
*G01V 3/08* (2006.01)          *G01R 33/00* (2006.01)
*G01R 33/10* (2006.01)

(21) Numéro de dépôt: **19169003.1**

(22) Date de dépôt: **12.04.2019**

(54) **PROCÉDÉ DE CALIBRATION D'UN RÉSEAU DE MAGNÉTOMÈTRES**

KALIBRIERUNGSVERFAHREN EINES MAGNETOMETERNETZWERKS

METHOD OF CALIBRATING A MAGNETOMETER NETWORK

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.04.2018 FR 1853319**

(43) Date de publication de la demande:
**23.10.2019 Bulletin 2019/43**

(73) Titulaire: **Commissariat à l'énergie atomique
et aux énergies alternatives
75015 Paris (FR)**

(72) Inventeurs:
• **VIAL, Franck**
  **38054 GRENOBLE Cedex 09 (FR)**
• **ALOUI, Saifeddine**
  **38054 GRENOBLE Cedex 09 (FR)**

(74) Mandataire: **Brevalex
95, rue d'Amsterdam
75378 Paris Cedex 8 (FR)**

(56) Documents cités:
**EP-A2- 1 184 684          WO-A1-2018/011492
WO-A2-2014/079740**

**Description**

**DOMAINE TECHNIQUE**

Le domaine de l'invention est celui du suivi du mouvement d'un objet magnétique mobile au moyen d'un ensemble de magnétomètres agencés en réseau. L'invention concerne plus particulièrement la calibration du réseau de magnétomètres pour connaître les positions et orientations des magnétomètres dans un repère associé au réseau.

**ÉTAT DE LA TECHNIQUE ANTÉRIEURE**

Le suivi du mouvement d'un ou plusieurs objets magnétiques peut être réalisé au moyen d'un ensemble de magnétomètres solidaires d'un même support mécanique de sorte que leurs positions et orientations relatives sont fixes dans le temps. Les magnétomètres sont ainsi agencés en réseau, ce réseau étant parfois abusivement qualifié de matriciel alors que les positions et orientations relatives des magnétomètres ne sont pas nécessairement ordonnées de manière régulière et que les magnétomètres ne sont pas nécessairement disposés dans un même plan. Par réseau de magnétomètres, on entend ici tout agencement de capteurs de champ magnétique, statique ou alternatif.

Afin d'estimer des paramètres de l'objet magnétique tels que sa position et son orientation dans un repère associé au réseau de magnétomètres, typiquement un repère solidaire du support portant les magnétomètres, on peut mettre en œuvre un procédé de localisation qui exploite :

- un modèle permettant de simuler le champ magnétique rayonné par l'objet en tout point de l'espace ;
- des mesures issues des magnétomètres du réseau ; et
- un algorithme capable d'estimer la position et l'orientation de l'objet magnétique en exploitant l'écart entre les mesures attendues et les mesures observées.

La mise en œuvre d'un tel procédé suppose de connaitre au préalable les positions et les orientations des magnétomètres dans le repère du réseau. Or cette connaissance peut être erronée du fait des tolérances de fabrication et/ou d'assemblage du réseau, ainsi que du fait des tolérances de fabrication des magnétomètres (la position et l'orientation réelles des axes de mesures d'un magnétomètre ne sont généralement pas fournies, ou quand elles le sont, c'est au mieux avec une tolérance de l'ordre de $500\,\mu m$ et 1° respectivement). Il apparaît donc primordial de procéder à une calibration des magnétomètres dans le repère du réseau (donc post-fabrication et post-assemblage) avant de pouvoir exploiter les mesures pour disposer d'une localisation suffisamment précise de l'objet magnétique.

A titre d'exemple de technique de calibration connue d'un réseau de magnétomètres, on peut citer le document FR 2 915 568 B1 qui divulgue un procédé qui permet de calibrer les sensibilités et les défauts d'orthogonalité des axes de mesure d'un ou plusieurs magnétomètres en effectuant des rotations dans un champ magnétique uniforme. La démarche peut être étendue à l'ensemble des magnétomètres d'un réseau à condition que le champ magnétique soit homogène et uniforme sur l'ensemble du volume occupé par le réseau au cours des rotations. Cette technique permet de remonter aux orientations des magnétomètres du réseau. En revanche, elle ne permet pas d'accéder à la position des magnétomètres au sein du réseau.

Un autre exemple de technique de calibration connue d'un réseau de magnétomètres est fourni par le document US 7,133,793 B2 qui propose de calibrer une dalle de magnétomètres (digitaliseur X-Y) destinée à localiser la position XY d'une source magnétique de type stylet. Le système de calibration est composé d'une grille de générateurs de champ (bobines) positionnée au-dessus de la dalle. L'alignement de la grille avec la dalle est mesuré par imagerie. Les générateurs, dont la position est connue au sein de la grille, sont activés séquentiellement. Un algorithme compare les positions connues des générateurs avec celles estimées par la dalle, de sorte à générer une carte de calibration. Cette carte est ensuite utilisée pour corriger les décalages de positionnement X-Y et les non linéarités de la dalle.

Dans ce document US 7,133,793 B2, la grille est utilisée comme référence et doit donc être parfaitement réalisée et/ou caractérisée. Sa maille et sa surface doivent en outre être adaptée à la surface de la dalle et à la résolution spatiale souhaitée. La calibration nécessite par ailleurs un système d'imagerie de positionnement ainsi qu'une électronique de pilotage des bobines. Cette technique de calibration apparaît donc complexe et onéreuse, pour ne traiter en outre qu'une localisation 2D. WO 2014/079740 A2 propose une technique de reconnaissance automatique d'un objet magnétique mobile qui vient déterminer des caractéristiques de l'objet au moyen d'un réseau de magnétomètres et les comparer à des caractéristiques pré-enregistrées dans une base de données.

**EXPOSÉ DE L'INVENTION**

L'invention a pour objectif d'améliorer la précision d'une localisation (position et/ou orientation) d'un objet mobile par un réseau de magnétomètres en proposant une technique simple, rapide et bas coût pour calibrer collectivement les magnétomètres du réseau avec un minimum d'équipements externes.

A cet effet, l'invention propose un procédé de calibration d'un réseau de magnétomètres, comportant les étapes suivantes :

- acquisition de mesures réalisées par les magnétomètres du réseau au cours d'un déplacement en survol du réseau d'un outil portant au moins deux sources de champ magnétique, dont un attribut est connu ;
- reconstruction, à partir des mesures acquises et pour une localisation possible des magnétomètres du réseau, d'une trajectoire des sources de champ magnétique au cours du déplacement ;
- calcul d'une estimation d'un attribut de l'outil à partir de la trajectoire reconstruite;
- comparaison entre l'attribut connu de l'outil et l'estimation calculée dudit attribut ; et
- détermination d'une localisation des magnétomètres du réseau à partir du résultat de l'étape de comparaison.

Certains aspects préférés mais non limitatifs de ce procédé sont les suivants :

- les étapes de reconstruction, de calcul et de comparaison sont réitérées pour une ou plusieurs autres localisations possibles des magnétomètres, la localisation déterminée lors de l'étape de détermination correspondant à une localisation possible pour laquelle le résultat de la comparaison est inférieur à un seuil ou à la localisation possible pour laquelle le résultat de la comparaison est minimal après un nombre donné de réitérations ;
- l'étape de reconstruction comprend, pour chaque instant d'échantillonnage des mesures acquises, l'évaluation d'une ou plusieurs caractérisations possibles des sources de champ magnétique, l'évaluation d'une caractérisation possible comprenant le calcul d'un écart entre les mesures acquises et une estimation du champ magnétique généré à ladite localisation possible des magnétomètres par les sources de champ magnétique présentant ladite caractérisation possible ;
- une caractérisation possible des sources de champ magnétique comprend des caractéristiques géométriques et magnétiques constituées des positions et des moments magnétiques des sources de champ magnétique ;
- l'attribut de l'outil est une distance entre sources de champ magnétique portées par l'outil ;
- l'attribut de l'outil est une amplitude du moment magnétique de chacune des sources de champ magnétique ;
- l'attribut de l'outil est une orientation relative des moments magnétiques de chacune des sources de champ magnétique ;
- l'amplitude du moment magnétique de chacune des sources de champ magnétique est identique ;
- l'outil comporte deux sources de champ magnétique dont les vecteurs directeurs de moment magnétique sont orientés selon des directions opposées ;
- le déplacement de l'outil comprend une pluralité de phases statiques ;
- le déplacement de l'outil est réalisé à main levée par un opérateur ;
- le réseau de magnétomètres est constitué de magnétomètres appartenant à une pluralité de structures de magnétomètres associées ensemble.

## BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

- les figures 1 et 2 sont des schémas représentant deux variantes d'un outil porte-aimants pouvant être utilisé dans le cadre de l'invention ;
- la figure 3 est un schéma illustrant la définition d'un repère associé au réseau de magnétomètres ;
- la figure 4 représente un repère associé au réseau et l'outil porte-aimants dont les coordonnées sont exprimées dans le repère associé au réseau ;
- la figure 5 est un ordinogramme illustrant l'enchaînement des étapes du procédé selon l'invention.

## EXPOSÉ DÉTAILLÉ DE MODES DE RÉALISATION PARTICULIERS

L'invention concerne un procédé de calibration collective de magnétomètres portés par un support mécanique et formant un réseau de magnétomètres. L'objectif de la calibration est de déterminer une localisation, c'est-à-dire une position et/ou une orientation, de chaque magnétomètre formant le réseau, dans un repère associé au réseau, par exemple un repère ayant pour origine l'un des magnétomètres.

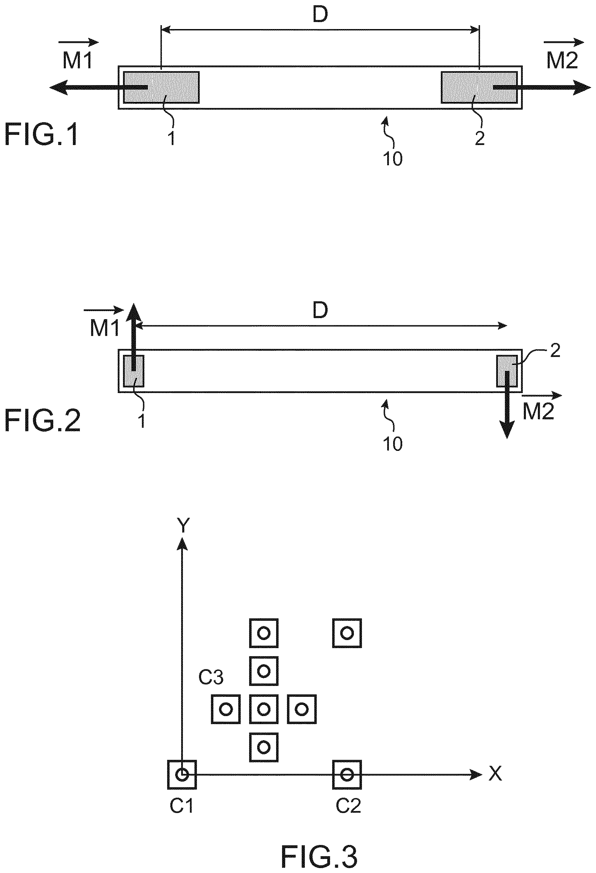

Le procédé de calibration selon l'invention exploite un outil portant au moins deux sources de champ magnétique, telles que des aimants ou des électroaimants dont un attribut (c'est-à-dire une caractéristique ou encore une propriété) est connu, par exemple un attribut géométrique ou magnétique. On prend dans ce qui suit l'exemple d'aimants et l'outil est dit porte-aimants. Comme représenté sur les figures 1 et 2, l'outil porte-aimants 10 peut prendre la forme d'un barreau en un matériau amagnétique, par exemple de l'aluminium, sur lequel sont fixés deux aimants 1, 2, avec typiquement un aimant agencé à chacune des extrémités du barreau. Bien entendu, l'outil porte-aimants 10 pourrait prendre toute autre forme, tel qu'un disque.

Un exemple d'attribut géométrique est celui de la distance relative D entre le barycentre des aimants 1, 2. On suppose que chacun de ces aimants 1, 2 est assimilable à un dipôle magnétique, et est donc ponctuel. Pour connaitre la distance relative D, il faut donc dans la pratique connaitre les dimensions des aimants et mesurer la distance entre les aimants, de manière à pouvoir en déduire la distance entre les deux barycentres.

Un exemple d'attribut magnétique est celui de l'amplitude des moments magnétiques de chacun des aimants. Un autre exemple d'attribut magnétique est celui de l'orientation relative des moments magnétiques des aimants 1, 2.

On prévoit de préférence que les amplitudes $m1$, $m2$ des moments magnétiques $\overrightarrow{M1} = m1\overrightarrow{u1}$, $\overrightarrow{M2} = m2\overrightarrow{u2}$ des aimants 1, 2 sont identiques afin de limiter les effets de masquage magnétique de l'un par rapport à l'autre. Par ailleurs, les vecteurs directeurs $\overrightarrow{u1}$, $\overrightarrow{u2}$ des moments magnétiques $\overrightarrow{M1}$, $\overrightarrow{M2}$ des aimants 1, 2 sont de préférence orientés selon des directions opposées de manière à accentuer les gradients magnétiques au niveau des magnétomètres du réseau et ainsi enrichir l'information. Ces vecteurs directeurs sont par exemple parallèles (figure 1) ou orthogonaux (figure 2) à l'axe longitudinal du barreau.

Le ou les attributs de l'outil porte-aimants 10, par exemple les amplitudes des moments magnétiques, l'orientation relative des moments magnétiques et la distance relative D, sont connus avec un niveau de précision cohérent avec celui de la calibration attendue. Pour une calibration fine, l'amplitude des moments magnétiques est connue typiquement à mieux que 0.5% près, leur orientation relative à mieux que 1° et la distance D à mieux que 100μm. Des précisions respectivement supérieures à 10%, 10° et 1mm sont jugées non suffisantes pour aboutir à une calibration qualitativement significative.

L'amplitude $m1$, $m2$ du moment magnétique des aimants 1, 2 qui dépend de la nature du matériau et du volume des aimants, est adaptée à la résolution des magnétomètres du réseau et à la portée visée, par exemple 0.3A.m$^2$ pour un déplacement de l'outil 10 à quelques centimètres au-dessus d'un réseau comportant des magnétomètres sensibles à quelques centaines de nano Tesla (0.3A.m$^2$ correspond à environ 300mm$^3$ de matériau magnétique Néodyme-Fer-Bore).

La distance D entre les aimants est suffisante pour que ces deux sources magnétiques soient séparables du point de vue des magnétomètres. Cette distance peut avantageusement être réduite en positionnant les aimants tête-bêche afin de générer des gradients magnétiques locaux forts. En toute rigueur, cette distance D ne doit pas être inférieure à la plus petite distance séparant deux magnétomètres du réseau. Cette distance D est telle qu'une projection des aimants 1, 2 sur le réseau, par exemple sur un plan horizontal si le réseau est à l'horizontal, est inscrite à l'intérieur de l'aire occupée par l'ensemble des magnétomètres formant le réseau. Elle peut typiquement être fixée à 10cm pour un survol à quelques centimètres de magnétomètres espacés de quelques centimètres et occupant la surface d'une feuille A4.

Une distance de survol trop faible et/ou un moment magnétique trop fort au regard de la dynamique de mesure des magnétomètres conduisent à un champ magnétique trop élevé et donc à la saturation des magnétomètres. A l'inverse un survol à une distance trop élevée et/ou un moment magnétique trop faible conduisent à un champ magnétique trop faible au regard du bruit de mesure des magnétomètres. Ainsi, on comprend que la distance de survol est choisie en fonction des performances de mesure des magnétomètres et du moment magnétique des aimants. En pratique, la valeur du champ au niveau des magnétomètres les plus proches des aimants doit être typiquement 300 à 3000 fois supérieure à leur bruit de mesure.

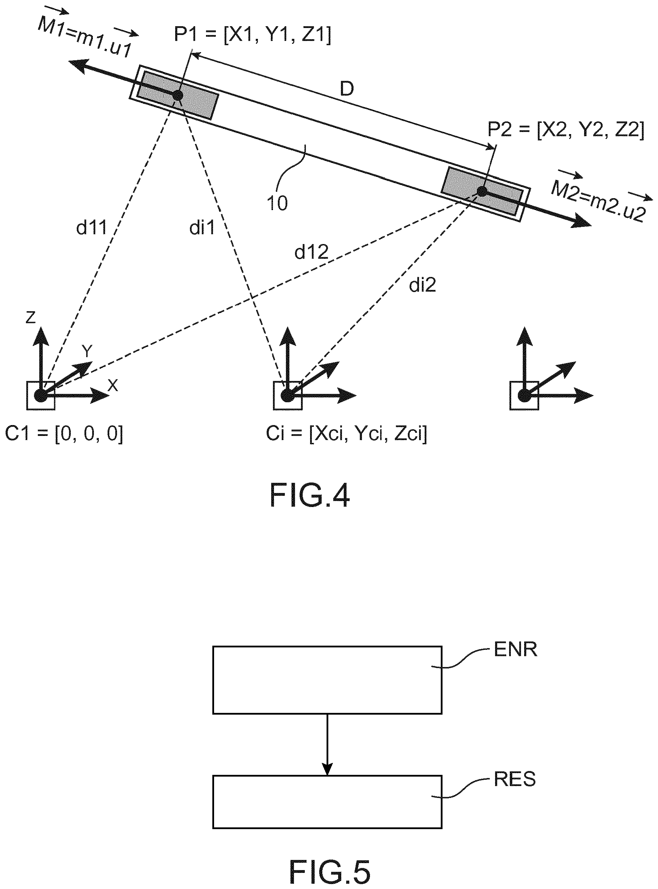

En référence à la figure 5, le procédé selon l'invention se décompose essentiellement en deux phases : une première phase ENR d'acquisition de mesures réalisées par les magnétomètres du réseau au cours d'un déplacement de l'outil 10 en survol du réseau de magnétomètres, et une deuxième phase RES au cours de laquelle les mesures acquises sont traitées. Ce procédé se résume comme suit.

L'outil porte-aimants 10 est déplacé au-dessus du réseau de magnétomètres, ceux-ci enregistrant l'évolution de la distribution du champ magnétique dont notamment celle générée par l'outil porte-aimants 10. Parallèlement, un modèle permet de décrire le comportement magnétique des aimants et de prédire la distribution spatiale du champ magnétique générée par le porte-aimants 10. En effet, en connaissant les caractéristiques géométriques et magnétiques des aimants, un modèle de prédiction peut être élaboré, comme cela sera décrit plus loin. En comparant les mesures effectives à des mesures prédites, la position et l'orientation des magnétomètres dans le repère du réseau peuvent être estimées.

On prend ici l'exemple d'un outil portant deux sources de champ magnétique 1, 2. L'invention n'est cependant pas limitée à cet exemple et s'étend à un outil portant plus que deux sources de champ magnétique, par exemple trois aimants agencés pour former un dièdre, voire une matrice d'aimants, ce qui peut s'avérer avantageux car les signaux enregistrés par les magnétomètres sont alors plus riches, avec un rapport signal à bruit logiquement plus favorable, ce qui permet de réduire le nombre de mesures et donc la durée de l'enregistrement. On dispose alors de plusieurs distances et de plusieurs angles (distances deux à deux et angles deux à deux). Par exemple, on peut disposer les aimants aux sommets de triangles, de telle manière que l'angle entre les moments magnétiques est de $2\pi/3$.

Phase d'acquisition ENR

L'outil 10 est déplacé au-dessus des magnétomètres qui chacun enregistre pendant ce temps l'évolution temporelle du champ magnétique perçu. Ce déplacement peut être effectué à main levée par un opérateur, de manière aléatoire. Toute la surface du réseau peut ainsi être balayée avec une hauteur de vol et une orientation de l'outil potentiellement variables. Les signaux sont d'autant plus riches en information que la trajectoire est variée. Les hauteurs de vol respectives des deux aimants sont de préférence maintenues proches l'une de l'autre de manière à ce qu'un aimant ne prenne pas le pas sur l'autre, les aimants générant un rapport signal à bruit similaire au niveau des magnétomètres. Autrement dit, l'outil 10 est déplacé de manière horizontale et, de préférence, en recouvrant successivement toutes les parties formant le réseau de magnétomètres dans un plan horizontal parallèle au réseau de magnétomètres.

La vitesse du déplacement doit être en adéquation avec la fréquence d'échantillonnage des magnétomètres, typiquement de l'ordre de 1cm/s pour une fréquence d'échantillonnage de 100Hz. Au total, le déplacement peut ainsi durer plusieurs dizaines de secondes.

Le déplacement peut aussi comprendre une pluralité de phases statiques, l'outil restant fixe pendant plusieurs secondes pour différentes positions au-dessus de la matrice. L'enregistrement est alors moins riche en termes d'information mais les phases statiques permettent de mieux filtrer le bruit de mesure. Pour ce faire, un support apte à porter l'outil peut être posé sur le réseau en différentes positions successives, prédéfinies ou non.

Phase de traitement RES

Cette phase RES comprend la résolution d'un problème d'optimisation pour déterminer une localisation des magnétomètres du réseau. Plus particulièrement, ladite localisation vient minimiser, au sens d'un critère d'optimisation, l'écart entre un attribut de l'outil 10 et une estimation de cet attribut calculée, sans connaissance du déplacement, à partir de ladite localisation et des mesures acquises lors de la phase d'acquisition ENR.

Par localisation venant minimiser l'écart au sens d'un critère d'optimisation, on entend typiquement une localisation associée à un écart inférieur à un seuil prédéterminé ou une localisation associée à un écart minimal après un nombre maximum d'itérations autorisées dépassé.

Dans un mode de réalisation possible, la localisation déterminée par la résolution du problème d'optimisation comprend simultanément les positions et orientations des magnétomètres du réseau.

Dans un autre mode de réalisation possible, le procédé comprend une étape préalable de détermination des orientations de chacun des magnétomètres du réseau, par exemple mise en œuvre selon la technique divulguée dans le document FR 2 9515 568 B1 discuté précédemment. La localisation déterminée par la résolution du problème d'optimisation comprend alors les positions des magnétomètres du réseau. Ce mode de réalisation permet de réduire le nombre de degrés de liberté du problème d'optimisation et ainsi d'éviter de conduire à une calibration erronée, notamment lorsque le rapport signal à bruit des mesures n'est pas favorable et/ou lorsque les mesures ne sont pas suffisamment fiables (par exemple du fait d'un biais variable ou de non-linéarités).

La résolution du problème d'optimisation comprend l'évaluation d'une pluralité de localisations possibles des magnétomètres du réseau. Chaque évaluation d'une localisation possible des magnétomètres du réseau comporte :

- à partir de la localisation possible et des mesures acquises, le calcul, sans connaissance du déplacement, d'une estimation de l'attribut de l'outil 10, et
- le calcul d'un écart entre l'attribut et l'estimation calculée dudit attribut.

Comme discuté précédemment, l'attribut connu de l'outil porte-aimants peut comprendre une distance D entre aimants 1, 2 de l'outil porte-aimants 10. En complément ou indépendamment, l'attribut connu peut comprendre une amplitude du moment magnétique de chacun des aimants de l'outil porte-aimants, ou encore leur orientation relative. On notera que l'estimation de l'attribut peut permettre d'identifier les aimants et donc, par exemple, de vérifier qu'il s'agit du bon outil 10.

Selon une approximation, chacun des aimants 1, 2 de l'outil porte-aimants 10 peut être assimilé à un dipôle magnétique ponctuel. Cette approximation devient valable si la distance entre un magnétomètre et l'aimant est au moins supérieure à deux fois la plus grande dimension de l'aimant (par exemple sa longueur pour un aimant cylindrique allongé, ou son diamètre pour un aimant en forme de pièce de monnaie). Les lois magnétostatiques permettent alors d'exprimer le champ magnétique en tout point de l'espace selon la formule :

$$B(P, u, m) \text{ en } \mu T = \frac{m 10^{-1}}{d^5}(3 < u, P > P - d^2 u)$$

Avec

$$P = \begin{bmatrix} x \\ y \\ z \end{bmatrix}$$

- $\quad$ la position de l'aimant par rapport au magnétomètre ;
- $\quad$ d = ‖P‖ la distance entre l'aimant et le magnétomètre, telle que $d^2 = x^2 + y^2 + z^2$;
- $\quad$ u est le vecteur directeur du moment magnétique de l'aimant ; et
- $\quad$ m est l'amplitude du moment magnétique de l'aimant.

On notera que des modèles magnétiques plus détaillés, comme par exemple le modèle ampérien décrit dans le document WO 2017/005915 A1, peuvent aussi être utilisés pour modéliser la distribution spatiale du champ magnétique généré par les deux aimants 1, 2, en particulier dans le cas où l'aimant passe très près des magnétomètres, l'approximation dipolaire n'étant alors plus valable.

Les coordonnées x, y, z sont ici exprimées dans le repère associé au réseau. Comme représenté sur la figure 3, ce repère peut être obtenu en choisissant, par exemple arbitrairement, un premier capteur C1 comme origine du repère, un deuxième capteur C2 pour définir un premier axe de référence, et un troisième capteur C3 pour définir un deuxième axe de référence, de sorte que ces deux axes de référence soient orthogonaux. Ce troisième capteur C3 permet de définir le sens du second axe mais n'est pas nécessairement aligné sur celui-ci. Un troisième axe peut être défini à l'aide d'un quatrième capteur, afin d'obtenir un repère orthogonal en trois dimensions pour un réseau de magnétomètres non plan.

La figure 4 représente l'outil porte-aimants 10 dont les coordonnées sont exprimées dans le repère associé au réseau, ayant pour origine le capteur C1. Pour un outil comportant deux aimants, le champ vu par chacun des magnétomètres est la somme des champs générés par chacun des deux aimants. Le champ vu par le capteur C1 se calcule comme suit :

$$B_{C1} = \frac{m_1.10^{-1}}{d_{11}^5}(3 < \mathbf{u_1}, \mathbf{P_1} > \mathbf{P_1} - d_{11}^5 \mathbf{u_1}) + \frac{m_2.10^{-1}}{d_{12}^5}(3 < \mathbf{u_2}, \mathbf{P_2} > \mathbf{P_2} - d_{12}^5 \mathbf{u_2}),$$

avec $d_{11} = ‖\boldsymbol{P}_1‖$ et $d_{12} = ‖\boldsymbol{P}_2‖$.

Le champ vu par le capteur Ci s'exprime comme suit :

$$B_{Ci} = \frac{m_1.10^{-1}}{d_{i1}^5}\big(3 < \mathbf{u_1}, (C_i - \mathbf{P_1}) > (C_i - \mathbf{P_1}) - d_{i1}^5 \mathbf{u_1}\big)$$

$$+ \frac{m_2.10^{-1}}{d_{i2}^5}\big(3 < \mathbf{u_2}, (C_i - \mathbf{P_2}) > (C_i - \mathbf{P_2}) - d_{i2}^5 \mathbf{u_2}\big)$$

avec $d_{i1} = ‖\boldsymbol{C_i} - \boldsymbol{P}_1‖$ et $d_{i2} = ‖\boldsymbol{C_i} - \boldsymbol{P}_2‖$.

La distance D entre les deux aimants se calcule comme $D = ‖\boldsymbol{P}_1 - \boldsymbol{P}_2‖$.

Le calcul, à partir des mesures acquises lors du déplacement de l'outil porte-aimants 10, de l'estimation de l'attribut de l'outil dépend de la connaissance des positions et orientations des divers magnétomètres du réseau. Ainsi une erreur portant sur cette connaissance des positions et orientations des magnétomètres se traduit par une erreur sur l'estimation de l'attribut et donc un écart entre la valeur réelle de l'attribut et son estimation.

Partant d'une localisation possible des magnétomètres, il est ainsi possible de déterminer une erreur d'estimation de l'attribut, par exemple une erreur d'estimation de la distance entre les aimants ou encore une erreur d'estimation de l'amplitude du moment magnétique de chaque aimant. Ainsi, l'invention permet de s'affranchir d'un référentiel extérieur au réseau pour estimer des erreurs de positionnement des magnétomètres du réseau et autorise une auto-calibration facile à mettre en œuvre à l'aide d'un outil porte-aimants 10.

Pour chaque instant d'échantillonnage, on peut ainsi calculer un vecteur d'erreur entre les attributs connus et l'estimation de ces attributs. On note *a* le vecteur des attributs connus et *â* le vecteur des attributs estimés pour la localisation possible des magnétomètres. Une erreur quadratique peut être calculée sur l'ensemble des n instants d'échantillonnage selon l'équation n°1 suivante : $\epsilon = \sum_{i=1}^{n} (\hat{a}(\text{loc}, [B_{0\ldots m-1}]_i) - a)^2$ , avec *loc* le vecteur contenant les paramètres de localisation que l'on cherche à trouver (positions et, le cas échéant, orientations des magnétomètres ; qui restent fixes au cours du temps), et $[B_{0\ldots m-1}]_i$ le vecteur des mesures magnétiques réalisées par les magnétomètres 0 à m-1 à l'instant d'échantillonnage i.

Ainsi, pour trouver le vecteur *loc,* on cherche à résoudre le problème d'optimisation consistant à minimiser cette erreur quadratique selon l'équation n°2 suivante.

$$loc = \underset{loc}{\operatorname{argmin}} \sum_{i=1}^{n} (\hat{a}(loc, [B_{0\ldots m-1}]_i) - a)^2.$$

Différents algorithmes d'optimisation peuvent être utilisés pour minimiser cette fonction. Par exemple, il est possible d'utiliser une descente de gradient sous réserve de dérivabilité, c'est à dire de pourvoir calculer une variation de *â* en fonction d'une variation de *loc.* Dans ce cas, la dérivée est calculée numériquement. Il est aussi possible d'utiliser un algorithme génétique qui permet de s'affranchir des problèmes liés au calcul de variation mentionné précédemment. Un algorithme hybride, utilisant des mutations aléatoires lorsqu'un problème de dérivabilité survient et une descente de gradient classique en l'absence de problèmes de dérivabilité, peut être préféré. La résolution de l'équation n°2 peut également être réalisée au moyen d'un filtre de Kalman, d'un filtre particulaire ou d'un réseau de neurones.

Le calcul du vecteur *â* des attributs estimés pour une localisation possible des magnétomètres peut être effectué en plusieurs étapes. Par exemple, si *â* porte sur la distance D entre les aimants, une première étape consiste à estimer la trajectoire suivie par les aimants lors du déplacement (les positions et moments magnétiques des deux aimants à chaque instant i) selon une technique similaire d'optimisation : on cherche les positions et moments magnétiques des aimants qui minimisent l'erreur, par exemple quadratique, entre les valeurs de champs magnétiques mesurées à l'instant i et celles estimées en fixant les positions et orientations des magnétomètres. Une fois les positions et moments magnétiques des aimants estimés sur l'ensemble des instants i, on calcule la distance D grâce à la formule donnée précédemment. Si le vecteur *â* porte sur les normes des deux moments magnétiques, l'étape mentionnée ci-dessus est effectuée, puis les normes sont déduites des valeurs des moments (vecteurs 3D) estimés. Si le vecteur *â* porte sur l'orientation relative des deux aimants, l'étape ci-dessus est effectuée, puis l'orientation relative est estimée par exemple à partir du produit scalaire entre les 2 vecteurs directeurs $\overrightarrow{u1}$ et $\overrightarrow{u2}$.

Ainsi, l'estimation de l'attribut de l'outil porte-aimants (i.e., le calcul du vecteur *â* pour une localisation possible des magnétomètres) est précédée d'une étape de reconstruction, à partir des mesures acquises et pour une localisation possible des magnétomètres du réseau, d'une trajectoire des aimants de l'outil porte-aimants au cours du déplacement.

Cette étape de reconstruction comprend, pour chaque instant d'échantillonnage des mesures acquises, l'évaluation d'une ou plusieurs caractérisations possibles des aimants de l'outil porte-aimants. Par caractérisation des aimants, on entend notamment les caractéristiques géométriques et magnétiques des aimants constituées par leurs positions et leurs moments magnétiques.

L'évaluation d'une caractérisation possible comprend le calcul d'un écart entre les mesures acquises et une estimation du champ magnétique généré à ladite localisation possible des magnétomètres par les aimants présentant ladite caractérisation possible. Puis, pour le calcul de l'estimation de l'attribut, on retient pour chacun des instants d'échantillonnage, la caractérisation possible associée à l'écart le plus faible.

Au global, il y a donc une optimisation récursive, à double étage : la première vise à estimer la trajectoire des aimants lors du déplacement de l'outil (les positions et moments magnétiques des aimants) pour une localisation des magnétomètres donnée (pour le calcul de *â* et de l'erreur $\epsilon$ selon l'équation n°1), la seconde vise à converger vers les positions et/ou orientations des magnétomètres qui minimisent l'erreur selon l'équation n°2. La première optimisation peut être réalisée selon l'un quelconque des algorithmes, filtres ou réseaux mentionnés précédemment en lien avec la seconde optimisation, sans nécessairement que les moyens mis en œuvre pour réaliser ces deux optimisations soient identiques. A titre d'exemple, un filtre de Kalman peut être mis en œuvre pour la première optimisation tandis qu'un algorithme de descente de gradient peut être mis en œuvre pour la seconde optimisation.

Ainsi, la phase de traitement RES comprend :

- une reconstruction, à partir des mesures acquises et pour une localisation possible des magnétomètres du réseau, d'une trajectoire des aimants de l'outil porte-aimants au cours du déplacement ;
- un calcul d'une estimation d'un attribut de l'outil porte-aimants à partir de la trajectoire reconstruite;
- une comparaison entre l'attribut de l'outil porte-aimants et l'estimation calculée dudit attribut ; et
- une détermination d'une localisation des magnétomètres du réseau à partir du résultat de la comparaison.

Les étapes de reconstruction, de calcul et de comparaison sont réitérées pour une ou plusieurs autres localisations possibles des magnétomètres jusqu'à ce que le résultat de l'étape de comparaison soit inférieur à un seuil. La localisation possible associée à cet égard minimal constitue alors la localisation optimale. Alternativement, la localisation possible est celle associée à l'écart minimal après un nombre donné de réitérations.

Dans tous les cas de figure, il est préférable d'initialiser l'algorithme avec le maximum de connaissances a priori, comme les positions et orientations relatives des magnétomètres (généralement, on sait par conception comment ils sont disposés dans le réseau), ainsi que leur sensibilité (données constructeur). L'algorithme est ainsi initialisé avec des données réalistes qu'il va affiner, ce qui facilite sa convergence.

La calibration réalisée par l'invention est simple et rapide. Elle ne nécessite ni d'équipements ni d'instruments externes lourds et couteux. Une calibration complète des magnétomètres (position, orientation et sensibilité) en une seule opération peut potentiellement être réalisée. L'invention peut par ailleurs être employée quelle que soit la distribution des magnétomètres, à condition que cette distribution reste fixe pendant et après la calibration (une connaissance a priori de cette distribution étant par ailleurs souhaitable pour faciliter la convergence de l'algorithme de localisation).

L'invention peut donc aussi être utilisée pour les systèmes à plusieurs structures de magnétomètres en venant exprimer les coordonnées de l'ensemble des magnétomètres dans un repère associé à l'une des structures prise comme référence. De tels systèmes comprennent par exemple des structures accolées en 2D ou 3D, de manière contiguë ou non, pour étendre le volume de mesure, densifier ce volume de capture ou changer la géométrie du volume de capture, comme par exemple des structures planes disposés en V. Dans un tel cas de figure, le réseau de magnétomètres au sens de l'invention est constitué de l'ensemble des magnétomètres des différentes structures associées ensemble.

L'invention n'est pas limitée au procédé tel que précédemment décrit, mais s'étend également à un dispositif de magnétométrie comprenant un réseau de magnétomètres et un calculateur configuré pour mettre en œuvre ce procédé ainsi qu'à un programme d'ordinateur comprenant des instructions qui, lorsque le programme est exécuté par ledit dispositif, conduisent celui-ci à mettre en œuvre ce procédé.

## Revendications

1. Procédé de calibration d'un réseau de magnétomètres (C1, C2, C3, Ci), **caractérisé en ce qu'**il comporte les étapes suivantes :

   - acquisition (ENR) de mesures réalisées par les magnétomètres du réseau au cours d'un déplacement en survol du réseau d'un outil portant au moins deux sources de champ magnétiques, dont un attribut (D, m1, m2) est connu ;
   - reconstruction, à partir des mesures acquises et pour une localisation possible des magnétomètres du réseau, d'une trajectoire des sources de champ magnétique au cours du déplacement ;
   - calcul d'une estimation d'un attribut (D, m1, m2) de l'outil à partir de la trajectoire reconstruite;
   - comparaison entre l'attribut connu de l'outil et l'estimation calculée dudit attribut ; et
   - détermination d'une localisation des magnétomètres du réseau à partir du résultat de l'étape de comparaison.

2. Procédé de calibration selon la revendication 1, dans lequel les étapes de reconstruction, de calcul et de comparaison sont réitérées pour une ou plusieurs autres localisations possibles des magnétomètres, la localisation déterminée lors de l'étape de détermination correspondant à une localisation possible pour laquelle le résultat de la comparaison est inférieur à un seuil ou à la localisation possible pour laquelle le résultat de la comparaison est minimal après un nombre donné de réitérations .

3. Procédé de calibration selon l'une des revendications 1 et 2, dans lequel l'étape de reconstruction comprend, pour chaque instant d'échantillonnage des mesures acquises, l'évaluation d'une ou plusieurs caractérisations possibles des sources de champ magnétique de l'outil, l'évaluation d'une caractérisation possible comprenant le calcul d'un écart entre les mesures acquises et une estimation du champ magnétique généré à ladite localisation possible des magnétomètres par les sources de champ magnétique présentant ladite caractérisation possible.

4. Procédé selon la revendication 3, dans lequel une caractérisation possible des sources de champ magnétique comprend des positions et des moments magnétiques des sources de champ magnétique.

5. Procédé de calibration selon l'une des revendications 1 à 4, dans lequel l'attribut de l'outil est une distance (D) entre sources de champ magnétique (1, 2) de l'outil (10).

6. Procédé de calibration selon l'une des revendications 1 à 4, dans lequel l'attribut de l'outil est une amplitude (m1, m2) du moment magnétique ($\overrightarrow{M1}$, $\overrightarrow{M2}$) de chacune des sources de champ magnétique (1, 2) de l'outil (10).

7. Procédé de calibration selon l'une des revendications 1 à 4, dans lequel l'attribut de l'outil est une orientation relative des moments magnétiques ($\overrightarrow{M1}$, $\overrightarrow{M2}$) de chacune des sources de champ magnétique (1, 2) de l'outil (10).

8. Procédé de calibration selon l'une des revendications 1 à 7, dans lequel l'amplitude (m1, m2) du moment magnétique de chacune des sources de champ magnétique (1, 2) de l'outil porte-aimants (10) est identique.

9. Procédé de calibration selon l'une des revendications 1 à 8, dans lequel l'outil (10) comporte deux sources de champ magnétique (1, 2) dont les vecteurs directeurs de moment magnétique ($\overrightarrow{u1}$, $\overrightarrow{u2}$) sont orientés selon des directions opposées.

10. Procédé selon l'une des revendications 1 à 9, dans lequel le déplacement de l'outil comprend une pluralité de phases statiques.

11. Procédé selon l'une des revendications 1 à 10, dans lequel le déplacement de l'outil est réalisé à main levée par un opérateur.

12. Procédé selon l'une des revendications 1 à 11, dans lequel le réseau de magnétomètres est constitué de magnétomètres appartenant à une pluralité de structures de magnétomètres associées ensemble.

13. Dispositif de magnétométrie comprenant un réseau de magnétomètres (C1, C2, C3, Ci) et un calculateur configuré pour mettre en œuvre le procédé de calibration selon l'une des revendications 1 à 12.

14. Programme d'ordinateur comprenant des instructions qui, lorsque le programme est exécuté par le dispositif selon la revendication 13, conduisent celui-ci à mettre en œuvre le procédé selon l'une des revendications 1 à 12.

**Patentansprüche**

1. Verfahren zum Kalibrieren eines Arrays von Magnetometern (C1, C2, C3, Ci), **dadurch gekennzeichnet, dass** es die folgenden Schritte umfasst:

   - Erfassen (ENR) von Messungen, die von den Magnetometern des Arrays während einer Fortbewegung durch Überfliegen des Arrays von einem Tool mit mindestens zwei Magnetfeldquellen durchgeführt werden, von denen ein Attribut (D, m1, m2) bekannt ist;
   - Rekonstruieren einer Flugbahn der Magnetfeldquellen während der Fortbewegung aus den erfassten Messungen und für einen möglichen Standort der Magnetometer des Arrays;
   - Berechnen einer Schätzung eines Attributs (D, m1, m2) des Tools anhand der rekonstruierten Flugbahn;
   - Vergleichen des bekannten Attributs des Tools mit der berechneten Schätzung dieses Attributs; und
   - Bestimmen eines Standorts der Magnetometer des Arrays anhand des Ergebnisses des Vergleichsschritts.

2. Kalibrierungsverfahren nach Anspruch 1, wobei die Schritte des Rekonstruierens, Berechnens und Vergleichens für einen oder mehrere andere mögliche Standorte der Magnetometer wiederholt werden, wobei der im Bestimmungsschritt ermittelte Standort einem möglichen Standort entspricht, für den das Ergebnis des Vergleichs unterhalb eines Schwellenwerts liegt, oder dem möglichen Standort, für den das Ergebnis des Vergleichs nach einer bestimmten Anzahl von Wiederholungen minimal ist.

3. Kalibrierungsverfahren nach einem der Ansprüche 1 und 2, wobei der Rekonstruktionsschritt für jede Abtastzeit der erfassten Messungen die Auswertung einer oder mehrerer möglicher Charakterisierungen der Magnetfeldquellen des Tools umfasst, wobei die Auswertung einer möglichen Charakterisierung die Berechnung einer Abweichung zwischen den erfassten Messungen und einer Schätzung des Magnetfelds umfasst, das an dem möglichen Standort der Magnetometer durch die Magnetfeldquellen erzeugt wird, die die mögliche Charakterisierung aufweisen.

4. Verfahren nach Anspruch 3, wobei eine mögliche Charakterisierung der Magnetfeldquellen Positionen und magnetische Momente der Magnetfeldquellen umfasst.

5. Kalibrierverfahren nach einem der Ansprüche 1 bis 4, wobei das Attribut des Tools ein Abstand (D) zwischen Magnetfeldquellen (1, 2) des Tools (10) ist.

6. Kalibrierverfahren nach einem der Ansprüche 1 bis 4, wobei das Toolattribut eine Amplitude (m1, m2) des magnetischen Moments ($\overrightarrow{M1}$, $\overrightarrow{M2}$) jeder der Magnetfeldquellen (1 , 2) des Tools (10) ist.

7. Kalibrierverfahren nach einem der Ansprüche 1 bis 4, wobei das Toolattribut eine relative Orientierung der magnetischen Momente ($\overrightarrow{M1}$, $\overrightarrow{M2}$) jeder der Magnetfeldquellen (1, 2) des Tools (10) ist.

8. Kalibrierverfahren nach einem der Ansprüche 1 bis 7, wobei die Amplitude (m1, m2) des magnetischen Moments jeder der Magnetfeldquellen (1, 2) des Magnetträgers (10) gleich ist.

9. Kalibrierverfahren nach einem der Ansprüche 1 bis 8, **dadurch gekennzeichnet, dass** das Tool (10) zwei Magnetfeldquellen (1, 2) umfasst, deren Magnetmoment-Richtungsvektoren ($\overrightarrow{u1}$, $\overrightarrow{u2}$) in entgegengesetzte Richtungen gerichtet sind.

10. Verfahren nach einem der Ansprüche 1 bis 9, wobei die Fortbewegung des Tools eine Vielzahl statischer Phasen umfasst.

11. Verfahren nach einem der Ansprüche 1 bis 10, wobei die Fortbewegung des Tools von einer Bedienungsperson freihändig ausgeführt wird.

12. Verfahren nach einem der Ansprüche 1 bis 11, wobei das Array von Magnetometern Magnetometer umfasst, die zu einer Vielzahl von miteinander verbundenen Magnetometerstrukturen gehören.

13. Magnetometervorrichtung mit einem Array von Magnetometern (C1, C2, C3, Ci) und einem Computer, der dazu ausgelegt ist, das Kalibrierungsverfahren nach einem der Ansprüche 1 bis 12 durchzuführen.

14. Computerprogramm mit Befehlen, die, wenn das Programm von der Vorrichtung nach Anspruch 13 ausgeführt wird, diese veranlassen, das Verfahren nach einem der Ansprüche 1 bis 12 durchzuführen.


**Claims**

1. A method for calibrating a network of magnetometers (C1, C2, C3, Ci), **characterized in that** it comprises the steps of:

    - acquiring (ENR) measurements made by the magnetometers of the network when a tool carrying at least two magnetic field sources, an attribute (D, m1, m2) of which being known, is displaced over the network of magnetometers;
    - reconstructing a path followed by the magnetic field sources during the displacement, using the acquired measurements and for a possible localisation of the magnetometers of the network;
    - calculating an estimate of a tool attribute (D, m1, m2) from the reconstructed path;
    - comparing the known tool attribute and the calculated estimate of said attribute; and
    - determining a localisation of the magnetometers of the network using the result of the comparing step.

2. Calibration method according to claim 1, in which the reconstructing, calculating and comparing steps are reiterated for one or several other possible localisations of the magnetometers, the localisation determined during the determining step corresponding to a possible localisation for which the result of the comparing step is less than a threshold or to a possible localisation for which the result of the comparing step is minimal after a given number of reiterations.

3. Calibration method according to one of claims 1 and 2, in which for each sampling instant of the acquired measurements, the reconstructing step comprises evaluating one or several possible characterisations of the magnetic field sources of the tool, evaluating a possible characterisation comprising calculating a difference between the acquired measurements and an estimate of the magnetic field generated at said possible localisation of the magnetometers by the magnetic field sources having said possible characterisation.

4. Method according to claim 3, in which a possible characterisation of the magnetic field sources includes positions and magnetic moments of the magnetic field sources.

5. Calibration method according to one of claims 1 to 4, in which the tool attribute is a distance (D) between the magnetic field sources (1, 2) of the tool (10).

6. Calibration method according to one of claims 1 to 4, in which the tool attribute is an amplitude (m1, m2) of the magnetic moment ($\overrightarrow{M1}$, $\overrightarrow{M2}$) of each of the magnetic field sources (1, 2) of the tool (10).

7. Calibration method according to one of claims 1 to 4, in which the tool attribute is a relative orientation of the magnetic moments ($\overrightarrow{M1}$), ($\overrightarrow{M2}$) of each of the magnetic field sources (1, 2) of the tool (10).

8. Calibration method according to one of claims 1 to 7, in which the amplitude (m1, m2) of the magnetic moment of each of the magnetic field sources (1, 2) of the magnet-holder tool (10) is identical.

9. Calibration method according to one of claims 1 to 8, in which the tool (10) comprises two magnetic field sources (1, 2) of which magnetic moment direction vectors (($\overrightarrow{u1}$), ($\overrightarrow{u2}$) are oriented according opposite directions.

10. Method according to one of claims 1 to 9, wherein the displacement of the tool comprises a plurality of static phases.

11. Method according to one of claims 1 to 10, in which the tool is displaced manually by an operator.

12. Method according to one of claims 1 to 11, in which the network of magnetometers is composed of magnetometers belonging to a plurality of magnetometer structures associated with each other.

13. Magnetometry device comprising a network of magnetometers (C1, C2, C3, Ci) and a calculator configured to implement the calibration method according to one of claims 1 to 12.

14. A computer program comprising instructions which, when the program is run on the device according to claim 13, cause the device to implement the method according to one of claims 1 to 12.

FIG.1

FIG.2

FIG.3

$\overrightarrow{M1} = m1.\overrightarrow{u1}$   P1 = [X1, Y1, Z1]

D

P2 = [X2, Y2, Z2]

10

$\overrightarrow{M2} = m2.\overrightarrow{u2}$

d11   di1

d12

di2

Z

Y

X

C1 = [0, 0, 0]   Ci = [Xci, Yci, Zci]

## FIG.4

ENR

RES

## FIG.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2915568 B1
- US 7133793 B2
- WO 2014079740 A2
- FR 29515568 B1
- WO 2017005915 A1